# EUROPÄISCHE PATENTSCHRIFT

(11) **EP 3 203 931 B1**
(45) Veröffentlichungstag und Bekanntmachung des Hinweises auf die Patenterteilung: **26.09.2018**
(21) Anmeldenummer: 15777934.9
(22) Anmeldetag: 08.10.2015
(51) Int. Cl.: A61F 2/07

(54) **GEFÄSSPROTHESEN-SYSTEM**
VASCULAR PROSTHESIS SYSTEM
SYSTÈME DE PROTHÈSE VASCULAIRE

(30) Priorität: 10.10.2014 DE 102014114747
(43) Veröffentlichungstag der Anmeldung: 16.08.2017
(73) Patentinhaber: JOTEC GmbH, 72379 Hechingen (DE)
(72) Erfinder: BARTHOLD, Franz-Peter, 72336 Balingen (DE); KLEIN, Karsten, 72116 Moessingen (DE); ODERMATT, Petra, 72379 Hechingen (DE); KLEINHANS, Severin, 72336 Balingen (DE)
(74) Vertreter: Witte, Weller & Partner Patentanwälte mbB
(86) Internationale Anmeldenummer: PCT/EP2015/073246
(87) Internationale Veröffentlichungsnummer: WO 2016/055564

(56) Entgegenhaltungen:
- DE-A1- 10 337 739
- DE-A1-102012 100 839
- DE-A1-102012 103 985
- DE-U1- 20 115 706
- US-A1- 2006 142 835

## Beschreibung

Die vorliegende Erfindung betrifft ein Gefäßprothesen-System zur Einführung in und Unterstützung eines Blutgefäß eines Patienten, welches Gefäßprothesen-System von einem komprimierten Zustand in einen expandierten Zustand überführbar ist.

Insbesondere betrifft die vorliegende ein Gefäßprothesen-System, das im Bereich des Aortenbogens implantiert wird.

Derartige Gefäßimplantate sind im Stand der Technik bspw. aus der DE 103 37 739.5 bekannt.

Allgemein ist es bekannt, intraluminale Gefäßprothesen, die auch als endovaskuläre Gefäßstents/-stentgrafts bezeichnet werden, zur Behandlung von geschwächten, verletzten, gerissenen oder aneurysmatischen Gefäßen einzusetzen. Hierfür wird an der erkrankten oder verletzten Stelle des Gefäßes eine Gefäßprothese bzw. ein Stentgraft freigesetzt, die/der die Funktionalität des ursprünglichen Gefäßes wieder herstellt bzw. die noch bestehende Gefäßintegrität unterstützt.

Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen, oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysma kann im fortgeschrittenen Stadium zu einer Ruptur der Arterie führen, mit der Folge, dass der Patient innerlich verblutet.

Die zur Behandlung derartiger Aneurysmen verwendeten selbstexpandierenden Gefäßimplantate bestehen im Allgemeinen aus einem hohlzylindrischen Metallrahmen bzw.-gerüst, dessen Mantelfläche mit einer Textil- oder Polymerfolie abgedeckt ist, so dass sich ein hohlzylindrischer Körper ergibt. Zur Implantation wird die Gefäßprothese radial zusammengedrückt, so dass sich seine Querschnittsfläche deutlich verringert. Die Gefäßprothese wird dann mit Hilfe eines Einführsystems in den Bereich des Aneurysma gebracht, wo es freigesetzt wird. Auf Grund der Federwirkung des Metallrahmens/-gerüsts expandiert die Gefäßprothese wieder in seine ursprüngliche Form und spannt dabei seine Mantelfläche auf, die sich proximal und distal von dem Aneurysma innen in dem Blutgefäß verklemmt. Auf diese Weise fließt das Blut jetzt durch die Gefäßprothese und eine weitere Belastung der Aussackung wird verhindert.

Der Metallrahmen solcher Gefäßprothesen besteht in der Regel bspw. aus einem Drahtgeflecht oder aus hintereinander angeordneten, mäanderförmig umlaufenden, sogenannten Stentfedern, die ggf. über Verbindungsstützen aus Draht miteinander verbunden sind, oder die lediglich über das Prothesenmaterial miteinander verbunden sind. Das Drahtgeflecht bzw. die Stentfedern sind üblicherweise aus einem Shape-Memory-Material, in der Regel aus Nitinol, wodurch die Stentfedern nach Einbringung in ein Gefäß zur Freisetzung wieder in den expandierten Zustand übergehen und das Gefäßimplantat dadurch "aufspannen".

Allgemein treten Aneurysmen häufig im Bereich der Baucharterie (Aorta abdominalis) oder Brustarterie (Aorta thoracica) auf. Zur Behandlung von Aneurysmen in der Bauch- oder Brustarterie ist es bereits bekannt, die Arterie durch Implantation eines Stents soweit zu stabilisieren, dass eine Ruptur des Gefäßes vermieden wird.

Aneurysmen können aber auch in dem sogenannten aufsteigenden Ast der Aorta (Aorta ascendens) auftreten. Der aufsteigende Ast der Aorta ist unmittelbar mit dem Herz verbunden. Ausgehend von der Aortenwurzel (Sinus aortae) verläuft der aufsteigende Ast in leicht gekrümmter Form vom Herz weg nach oben und geht dort in den Aortenbogen (Arcus aortae) über. Im Bereich des Aortenbogens zweigen die Kopfgefäße ab, u.a. die linke und die rechte Halsschlagader. Der Aortenbogen weist einen Kurvenverlauf von etwa 180° mit einem sehr engen Radius auf und verbindet den aufsteigenden Ast der Aorta mit der Brustarterie und im weiteren Verlauf mit der Baucharterie.

Nicht nur im Bereich des Aortenbogens ist es wichtig, vom Hauptgefäße abgehende Seiten-Gefäße durch die Positionierung der Gefäßprothese nicht zu blockieren, weshalb viele Gefäßprothesen offene Zonen oder sogenannte Fenestrierungen aufweisen, über welche vom Gefäßimplantat abgehende Seitenäste, die in die Seiten-Gefäße hineinragen, eingeführt und am Gefäßimplantat fixiert werden können.

Ein Aneurysma oder eine Dissektion im aufsteigenden Ast der Aorta wird bis heute in der Regel in einem invasiven offenen Eingriff behandelt. Eine solche Operation macht bisher regelmäßig zwei große, zeitlich getrennte Eingriffe erforderlich, und stellt einen sehr großen und aufwändigen und damit gefährlichen Eingriff dar, da nicht nur das Herz, sondern auch das Gehirn und die Bauchorgane des Patienten einer hypothermen Perfusion, d.h. also einer künstlichen, kalten extrakorporalen Durchblutung, bzw. einem hypothermen Durchblutungsstillstand unterzogen werden müssen. Mit einem solchen Eingriff sind jedoch nur wenige Herzchirurgen an erfahrenen Zentren vertraut.

Nach wie vor besteht daher ein großes Bedürfnis nach Stent-/Stentgraftsystemen, bzw Gefäßprothesen mit Hilfe derer der oben geschilderte Eingriff vereinfacht und zeitlich verkürzt werden könnte.

Bisher ist im Stand der Technik kein Stent- oder Stentgraftsystem bekannt, mit Hilfe dessen der oben geschilderte Eingriff vereinfacht und zeitlich verkürzt werden könnte, weshalb nach wie vor ein großes Bedürfnis nach einem solchen System besteht.

Aufgabe der vorliegenden Erfindung ist es daher, ein System bereitzustellen, mit welchem der Bereich der aufsteigenden Aorta, des Aortenbogens und der absteigendem Aorta schnell und unkompliziert behandelt werden kann, bzw. das die oben geschilderten Eingriffe auch weniger erfahrenen Herzchirurgen ermöglicht.

Gemäß der vorliegenden Erfindung wird diese Aufgabe gelöst durch ein Gefäßprothesen-System zur Einführung in und Unterstützung eines Blutgefäß eines Patienten, welches Gefäßprothesen-System von einem komprimierten Zustand in einen expandierten Zustand überführbar ist, wobei das Gefäßprothesen-System i) ein Stentgraft-Element mit einem hohlzylindrischen Körper, einem ersten, proximalen Stentgraft-Element-Ende und einem zweiten, distalen Stentgraft-Element -Ende aufweist, wobei das Stentgraft-Element mäanderförmig umlaufenden Stützen und ein an den Stützen befestigtes und diese verbindendes Prothesenmaterial aufweist zur Ausbildung eines umfänglich gecoverten Stentgraft-Elements, und ii) ein Stent-Element mit einem hohlzylindrischen Körper, einem ersten, proximalen Stent-Element-Ende und einem zweiten, distalen Stent-Element-Ende, wobei das Stent-Element ein Stent-Stützgerüst aufweist, das frei von Prothesenmaterial ist, zur Ausbildung eines ungecoverten Stent-Elements; ferner sind bei dem erfindungsgemäßen Gefäßprothesen-System das Stentgraft-Element und das Stent-Element zwei baulich voneinander getrennte Elemente des Gefäßprothesen-Systems, und das Stent-Element ist derart dimensioniert und ausgebildet, dass es mit seinem zweiten, distalen Stent-Element-Ende zumindest teilweise über das erste, proximale Stentgraft-Element-Ende in dieses einführbar und in diesem expandierbar ist; dabei ist ferner das Stent-Element an seinem ersten, proximalen Ende umlaufend mit einem stentfreien Prothesen-Abschnitt fest verbunden, welcher Prothesen-Abschnitt lediglich aus Prothesenmaterial besteht, und das Stentgraft-Element und das Stent-Element sind nur über einen streifenförmigen Prothesenmaterial-Abschnitt miteinander verbunden, derart, dass der streifenförmige Prothesenmaterial-Abschnitt mit einem ersten Ende am ersten, proximalen Stentgraft-Element-Ende und mit seinem zweiten Ende am ersten, proximalen Stent-Element-Ende zur Ausbildung einer Prothesenmaterial-Brücke fixiert ist.

Die der Erfindung zugrunde liegende Aufgabe wird auf diese Weise vollkommen gelöst.

Mit dem neuen Gefäßprothesen-System wird erreicht, dass auf einfache Weise ein von Prothesenmaterial-freier Abschnitt bereitgestellt werden kann, über welche die abgehenden Gefäße weiterhin mit Blut versorgt werden können, wobei gleichzeitig die verletzten betroffenen Gefäße durch den sogenannten gecoverten Stentgraft-Abschnitt, also den Abschnitt, der Prothesenmaterial aufweist, durch diese gestützt werden. Die Einführung und Platzierung dieses Systems kann durch dessen zweiteiligen Aufbau präzise und einfach gehandhabt werden. Durch die Verbindung des Stentgraft-Elements mit dem Stent-Element "nur", d.h. lediglich über einen streifenförmigen Prothesenmaterial-Abschnitt sind beide Elemente, d.h. das Stentgraft-Element und das Stent-Element relativ insbesondere im ungeladenen Zustand frei beweglich und können auch getrennt voneinander komprimiert werden. Das erfindungsgemäße Gefäßprothesen-System bietet daher den Vorteil, dass es in seiner Längsrichtung und Biegung den jeweiligen anatomischen Verhältnissen des zu behandelnden Patienten angeglichen werden kann.

Dabei bedeutet "nur" über einen streifenförmigen Prothesenmaterial-Abschnitt, dass die Stentgraft-Element und das Stent-Element außer der streifen förmigen Verbindung über ein Prothesenmaterial nicht miteinander verbunden sind. Dies bedeutet, dass die beiden Elemente voneinander getrennt hergestellt sind, und auch getrennt voneinander gehandhabt werden können, wobei der streifenförmige Prothesenmaterial-Abschnitt für eine Brücke zwischen den ansonsten unverbundenen Elementen sorgt. Durch diese fast freie Beweglichkeit des Stent-Elements gegenüber dem Stentgraft-Element wird erreicht, dass das Stent-Element unabhängig vom Stentgraft-Element und zumindest teilweise innerhalb des Stentgraft-Elements expandiert werden kann. Ferner wird dadurch eine Flexibilität des Gefäßprothesen-Systems erreicht, welche es ermöglicht, das Stent-Element stark gebogen frei zusetzen, ohne das Stentgraft-Element wesentlich zu beeinflussen, wobei durch den streifenförmigen Prothesenmaterial-Abschnitt dennoch eine Verbindung zwischen den beiden Elementen erreicht wird, durch welche eine Verrutschen oder Herausrutschen des Stent-Elements aus dem Stentgraft-Elements erreicht werden kann. Ferner wird durch diese Brücke das Stent-Element nach dem Freisetzen in eine Biegung gezwungen, wodurch es möglich ist, einen Prothesenmaterial-freien Stent-Abschnitt zu erhalten, der in Länge und Kurvatur individuell anpassbar ist.

Zur Einführung des Gefäßprothesen-Systems in ein Gefäß eines zu behandelnden Patienten wird dieses komprimiert, wobei das Stent-Element und das Stentgraft-Element von jeweils einer eigenen Hülle komprimiert werden, die unabhängig voneinander zurückgezogen werden können, um zum einen und zunächst das Stentgraft-Element freizusetzen und um zum anderen, und in einem zweiten Schritt, der unabhängig vom ersten durchgeführt werden kann, das Stent-Element freizusetzen. Dementsprechend sind beide Elemente konzentrisch auf einem Einführkatheter geladen, wobei das Stent-Element, komprimiert von einer eigenen Stent-Element-Hülle, innerhalb des ebenfalls von einer weiteren Hülle komprimierten Stentgraft-Elements zu liegen kommt.

Zur Freisetzung wird also zunächst, bspw. durch Kontrolle über entsprechende Marker am Gefäßprothesen-System, der Einführkatheter mit dem darauf geladenen System in das zu behandelnde Gefäß eingeführt und derart korrekt platziert und durch Rückziehen der Stentgraft-Element-Hülle expandiert, so dass es vom Hauptgefäß abgehende Gefäße nicht blockiert. In einem zweiten Schritt wird dann das Stent-Element durch Rückziehen der das Stent-Element komprimierenden Hülle zumindest teilweise innerhalb des Stentgraft-Elements freigesetzt, wobei das Stent-Element sich mit zumindest einem Abschnitt außerhalb des Stentgraft-Elements befindet, und dieser Abschnitt dann in dem Bereich der abgehenden Gefäße zu liegen kommt. Dies wird auch dadurch erreicht, dass sich das Stent-Element biegen kann, wobei der streifenförmige Prothesenmaterial-Abschnitt nicht im Bereich der abgehenden Gefäße, sondern an der gegenüber liegenden Gefäßwand zu liegen kommt.

Es ist also nicht notwendig, zwei getrennte Stentgraft- bzw. Stent-Elemente getrennt voneinander und mit zwei unterschiedlichen Einführkathetem einzuführen, sondern das erfindungsgemäße System kann mit einem Einführsystem bzw. - katheter eingeführt werden. Über das streifenförmige Prothesenmaterial, das die Brücke zwischen Stentgraft-Element und Stent-Element bildet, wird aber gleichzeitig sichergestellt, dass das Stent-Element nicht aus dem Stentgraft-Element vollständig herausrutscht: dadurch, dass das Stent-Element teilweise im Stentgraft-Element expandiert, wird ersteres im Inneren des Stentgraft-Elements verankert, bleibet aber zumindest mit dem nicht im Stentgraft-Element freigesetzten Abschnitt im Hinblick auf eine Biegung und Anpassung an bspw. den Aortenbogen flexibel. Mit dem außerdem am Stent-Element vorgesehenen stentfreien Prothesen-Abschnitt, der vorzugsweise kragenartig am anderen Ende des Stent-Elements vorgesehen ist, kann dann das Gefäßprothesen-System an der Gefäßwand vernäht und die Gefäßprothese insgesamt dadurch mit der Gefäßwand durch eine einzige Naht verbunden werden. Dies stellt einen erheblichen Vorteil dar, da sich die Dauer dieser Naht direkt auf die Dauer des chirurgischen Eingriffs und bspw. auch auf die Dauer der Kopfperfusion auswirkt.

Durch das erfindungsgemäße Stentgraft-System, also durch die Kombination zweier einzelnen, baulich voneinander getrennten, aber über die Brücke verbundenen Elemente, wird es dadurch auch bzw. beispielsweise bei einer Implantation in den Aortenbogen möglich, die drei Abschnitte der Aorta, also die Aorta ascendens (aufsteigenden Hauptschlagader), des Aortenbogens und der descendierenden Aorta (absteigenden Hauptschlagader) simultan zu behandeln. Damit kann auf die Resektion des Aortenbogens mit all den damit verbundenen komplexen Perfusionsanforderungen für Gehirn und unteren Körper verzichtet werden, und der Eingriff nur mit Durchtrennung des obersten Abschnitts der aufsteigenden Aorta in einer kurzen, ca. 10 -20 minütigen selektiven Kopfperfusionsphase oder hypothermen Stillstandsphase erfolgen, um die neue Gefäßprothese einzuführen und freizusetzen. Die Einführung und Freisetzung kann dabei einfach unter Sicht des Auges bzw. eines Angioskops erfolgen.

Gegenüber bisher im Stand der Technik bekannten Gefäßprothesensystemen für den Aortenbogen überwindet das erfindungsgemäße Gefäßprothesen-System ferner den Nachteil, dass bei der Freisetzung eine gewisse Flexibilität hinsichtlich der Landungszonen besteht, die insbesondere bei einteiligen Gefäßprothesen kritisch beachtet werden muss.

Erfindungsgemäß wird damit eine intraluminale Gefäßprothese bereitgestellt, mit welcher die Möglichkeit geschaffen wird, Eingriffe insbesondere am Aortenbogen, bzw. in der aufsteigenden Aorta, dem Aortenbogen und der absteigenden Aorta, chirurgisch zu vereinfachen und zeitlich deutlich zu verkürzen. Vorteilhafterweise können damit nicht nur hoch spezialisierte Herzchirurgen die weiter oben geschilderten Eingriffe am Aortenbogen vornehmen. Ferner kann die erfindungsgemäße Gefäßprothese auch bei schwer erkrankten Patienten und bei älteren Patienten mit bspw. altersbedingt beschädigten Aortenwandschichten und mit Nicht-Durchblutung von lebenswichtigen Organsystemen wie Gehirn oder Bauchorganen eingesetzt werden, da diese mit einem zeitlich verkürzten und chirurgisch vereinfachten Verfahren eingesetzt werden können.

Vorteilhafterweise wird dabei das von Prothesenmaterial freie Stent-Element im expandierten Zustand im Bereich des Aortenbogens freigesetzt. Dadurch wird gewährleistet, dass der Blutfluss in die abgehenden Gefäße, wie den Truncuns brachiocephalicus, die linke Aorta carotis communis, und die linke Arteria subclavia, nicht beeinträchtigt wird. Das durch den Aortenbogen und die hierin zu verankernde Gefäßprothesen -System fließende Blut kann diese durch die im ungecoverten Stent-Element vorliegenden Öffnungen der Gefäßprothese verlassen.

Vorzugsweise erstreckt sich der streifenförmige Prothesenmaterial-Abschnitt, der definitionsgemäß stentfrei ist, sich somit vom ersten, proximalen Stentgraft-Element-Ende zum ersten, proximalen Stent-Element-Ende.

Es versteht sich, dass zur Ausbildung der Prothesenmaterial-Brücke der streifenförmige Prothesenmaterial-Abschnitt lediglich an seinen beiden Enden mit der Gefäßprothese wie oben definiert fixiert, so dass sein zwischen den beiden Enden liegender Abschnitt frei bzw. nicht-fixiert auf der Gefäßprothese, d.h. auf dem Stent-Element, aufliegt.

Nachstehend werden einige hierin verwendeten Begriffe, die zwar für den Fachmann an sich und unter Heranziehung der vorliegenden Offenbarung klar sind, näher definiert:

Vorliegend wird unter einer "Stent-Feder", wie eingangs diskutiert, jedes einstückige, ringförmige Element verstanden, das sich aufgrund dessen Materials komprimieren lässt, und sich nach Entfernung des Kompressionsdrucks wieder federartig expandieren kann. Unter "mäanderförmig" wird vorliegen jeder schlingen- bzw. schleifenförmige Verlauf' der Stentfeder bzw. des Stentdrahtes verstanden, wobei jede Stentfeder einstückig, d.h. aus einem mäanderförmig umlaufenden Stentfederring gebildet ist.

Eine "mäanderförmig umlaufende einstückige Stentfeder" ist in diesem Zusammenhang vorliegend ein sich federartig expandierendes und komprimierbares ringförmiges Stentelement, das einen wellenartigen Umlauf besitzt, wobei sich Wellenberg und Wellental, die eine Phase bilden, abwechseln.

Vorteilhafterweise ist dabei ein Spitzbogen jeweils aus zwei Schenkeln und einem zwischen den Schenkeln liegenden Scheitelpunkt bzw. Tiefstpunkt gebildet.

"Zumindest ein Spitzbogen" bedeutet vorliegend, dass der Seitenkörper durch die Ausstellung eines einzigen Spitzbogens einer Stentfeder aufgespannt wird, oder aber durch zwei oder mehr Spitzbögen. In einer bevorzugten Ausführungsform sind zwei Spitzbögen einer Stentfeder ausgestellt. Grundsätzlich bildet die Mehrzahl der mäanderförmig umlaufenden Spitzbögen die Stentfeder des Grundkörpers, und der bzw. die ausgestellte(n) Spitzbogen/Spitzbögen bildet die Abzweigungsstelle für den Seitenkörper.

Grundsätzlich werden bei Stentgrafts oder endoluminalen Prothesen allgemein sowie vorliegend zur Bezeichnung der jeweiligen Enden die Begriffe "distal" und "proximal" verwendet, wobei der Begriff "distal" der Teil bzw. das Ende bezeichnet wird, der/das in Bezug auf den Blutstrom weiter stromabwärts liegt. Der Begriff "proximal" hingegen bezeichnet, wieder in Bezug auf den Blutstrom, einen Teil bzw. das Ende, der/das in Bezug auf den Blutstrom weiter stromaufwärts liegt. Anders ausgedrückt bedeutet der Begriff "distal" in Richtung des Blutstroms, und der Begriff "proximal" der Richtung des Blutstroms entgegengesetzt. Bei Kathetern hingegen, bzw. Einführsystemen bezeichnet der Begriff "distal" das Ende des Katheters bzw. Einführsystems, das in den Patienten eingeführt wird, bzw. das vom Anwender ausgesehen am Entferntesten liegt, und der Begriff "proximal" das Ende, das sich dem Anwender näher zugewandt ist.

Entsprechend sind vorliegend die "proximale" und die "distale" Öffnung des Gefäßimplantats die Öffnungen, durch die hindurch der Blutfluss durch den hohlzylindrischen Körper der Gefäßimplantat gewährleistet wird: Wenn das erfindungsgemäße Gefäßimplantat in einem Blutgefäß wie beispielsweise der Aorta implantiert ist, fließt also das vom Herzen kommende Blut durch die proximale Öffnung des Gefäßimplantats, und verlässt das Gefäßimplantat durch deren distale Öffnungen.

Das Gefäßprothesen-System bzw. dessen beide hohlzylindrische Elemente können dabei über ihre Gesamtlänge hinweg einen einheitlichen Durchmesser, oder aber unterschiedliche Durchmesser aufweisen.

Definitionsgemäß sind die Stentfedern nicht direkt miteinander verbunden, und weisen untereinander keine verbindenden Schenkel oder Streben oder ähnliche Verbindungselemente auf. Die Stentfedern sind lediglich über das Prothesenmaterial, an welches die Stentfedern angebracht sind, miteinander verbunden, wodurch eine "indirekte Verbindung" zwischen den Stentfedern geschaffen wird.

Vorliegend wird unter "Stent" bzw. "Stent-Stützgerüst" jede Vorrichtung oder eine Struktur bezeichnet, die einer Prothese eine Expansionskraft und/oder eine stützende Funktion verleiht. Entsprechend ist ein Stent-Element daher jedes Element, das die Eigenschaften eines Stents aufweist.

Der Ausdruck "Stentgraft" soll vorliegend - wie auch im Stand der Technik - eine Prothese bedeuten, die einen bzw. mehrere Stent (oder Stentfedern) sowie ein damit verbundenes Prothesen("graft")-Material aufweist, das ein Lumen durch zumindest einen Abschnitt der Prothese bildet. Ein solcher Stentgraft wird auch als gecoverter Stent(abschnitt) bezeichnet.

Ferner wird unter "streifenförmig" bzw. "streifenförmiger Prothesenmaterial-Abschnitt" jedes eher längliche als breite Element aus Prothesenmaterial verstanden, das eine Brücken- oder Latzartige Verbindung zwischen dem Stentgraft-Element und dem Stent-Element darstellt. Der streifenförmige Prothesenmaterial-Abschnitt ist dabei eine Art Verlängerung des Prothesenmaterials des Stentgraft-Abschnitts, wobei das streifenförmige Prothesenmaterial definitionsgemäß nicht über den gesamten Umfang des hohlzylindrischen Körpers des Stentgraft-Abschnitts ausgebildet ist, sondern nur über einen vergleichswiese kleineren Teil des Umfangs, eben zur Ausbildung eines streifenförmigen Abschnitts.

Gemäß einer Ausführungsform des erfindungsgemäßen Gefäßprothesen-Systems ist der stentfreie Prothesen-Abschnitt am ersten proximalen Stent-Element-Ende kragenförmig ausgebildet.

Gemäß einer weiteren Ausführungsform ist das erfindungsgemäße Gefäßprothesen-System, wie weiter oben erwähnt, zur Implantation in eine Aorta, insbesondere im Bereich der aufsteigenden Hauptschlagader, des Aortenbogens und der absteigenden Hauptschlagader ausgebildet ist. Dabei ist das Gefäßprothesen-System zur Einbringung in die Aorta von einem komprimierten Zustand in einen expandierten Zustand überführbar, und das Stentgraft-Element und das Stent-Element sind zur Verankerung des Gefäßprothesen-Systems in der Aorta ausgebildet.

In diesem Zusammenhang ist in weiteren Ausführungsformen vorgesehen und bevorzugt, wenn das von Prothesenmaterial freie Stent-Element im expandierten Zustand im Bereich des Aortenbogens freisetzbar ist.

Bei dem erfindungsgemäßen Gefäßprothesen-System ist in diesem Zusammenhang vorgesehen, wenn das gecoverte Stentgraft-Element distal der Arteria subclavia zu liegen kommt, und das nicht-gecoverte Stent-Element im Aortenbogen. Der stentfreie Prothesenabschnitt wird im proximalen Aortenbogen platziert und fixiert.

Anders ausgedrückt befindet sich daher das Stentgraft-Element im freigesetzten Zustand am distalen Endbereich des Gefäßprothesen-Systems und kann daher in der freigesetzten Form der beiden Elemente auch als distaler Gefäßprothesenabschnitt bezeichnet werden.

Im freigesetzten Zustand, in dem die das Stent-Element und das Stentgraft-Element durch ein teilweises Überlappen miteinander bzw. ineinander verankert sind, bilden das gecoverten Stentgraft-Element und der stentfreie Prothesenmaterial-Abschnitt also die äußeren Enden des erfindungsgemäßen Gefäßprothesen-Systems..

Gemäß einer weiteren Ausführungsform weisen zumindest die Stützten des Stentgrafts-Elements und/oder das Stent-Stützgerüst des Stent-Elements aus einem selbst-expandierenden Material sind oder ein solches auf. Hierbei ist insbesondere bevorzugt, wenn das Material Nitinol ist.

Wie bereits weiter oben diskutiert, ist in einer weiteren Ausführungsform ist das von Prothesenmaterial freie Stent-Element im expandierten Zustand im Bereich des Aortenbogens freisetzbar.

Bei dem erfindungsgemäßen Gefäßprothesen-System ist ferner in einer Ausführungsform bevorzugt, wenn das Stentgraft-Element zwischen zwei und acht hintereinander angeordnete Stützen aufweist.

Insbesondere ist bevorzugt, wenn zwei, drei, vier, fünf, sechs, sieben oder acht, hintereinander angeordnete mäanderförmig umlaufende Stützen vorgesehen sind. Gemäß einer bevorzugten Ausführungsform sind die Stützen bzw. Stentfedern selber untereinander nicht durch Stege o.ä. miteinander verbunden, bzw. stehen nicht in unmittelbaren Kontakt miteinander, sondern sind nur über das Prothesenmaterial zu einem hohlzylindrischen Körper verbunden. Diese Ausführungsform hat den Vorteil, dass der Fachmann die Länge des Stentgraftabschnitts den jeweiligen Gefäßgegebenheiten anpassen kann.

Gemäß einer weiteren Ausführungsform ist bei dem erfindungsgemäßen Gefäßprothesen-System bevorzugt, wenn das von Prothesenmaterial freie Stent-Element ein geflochtenes, gezwirbeltes oder Laser-geschnittenes Stent-Stützgerüst aufweist.

Dabei wird unter "Stent-Stützgerüst" jede Ausbildung eines Stents verstanden, bei der verschiedene Draht-Stränge derart miteinander verflochten, ineinander verschlungen oder anderweitig verknüpft sind, dass sich eine Struktur mit Zonen, Bereichen oder Punkten bildet, an denen die Stränge übereinander liegen, und mit Zonen oder Bereichen, die frei von den Drahtsträngen sind und daher Öffnungen oder Fenster oder Maschen bilden. Entsprechend weist auch ein Laser-geschnittenes Stent-Stützgerüst Maschen oder Öffnungen auf, über die das in der Aorta bzw. im Aortenbogen geführte Blut die Aorta in die abgehenden Gefäße, insbesondere der Truncus brachiocephalicus, die linke Aorta carotis communis, und die linke Arteria subclavia, verlassen kann und damit eine Durchblutung dieser Gefäße gewährleistet.

Bei dem erfindungsgemäßen Gefäßprothesen-System ist ferner in einer weiteren Ausführungsform bevorzugt, wenn der streifenförmige Prothesenmaterial-Abschnitt das Stentgraft-Element und das Stent-Element nur auf einem Kreisbogen von kleiner gleich 180° verbindet.

Wie weiter oben diskutiert, ist die Brücke, die das streifenförmige Prothesenmaterial bereitstellt, nicht über den gesamten Umfang gebildet. Dies hat den Vorteil, dass die Brücke nicht weite Teile des ungecoverten Stent-Elements abdeckt, sondern diese freilässt, um einen ungehinderten Blutfluss durch die Öffnungen im Stent-Element zuzulassen, aber gleichzeitig für eine Verbindung zwischen Stentgraft-Element und Stent-Element sorgt. Durch diese Brücke wird ferner das Stent-Element nach dem Freisetzen in eine Biegung gezwungen, wodurch es möglich ist, einen Prothesenmaterialfreien Stent-Abschnitt zu erhalten, der in Länge und Kurvatur individuell anpassbar ist.

Entsprechend ist gemäß einer bevorzugten Ausführungsform des erfindungsgemäßen Gefäßprothesen-Systems bevorzugt, wenn der streifenförmige Prothesenmaterial-Abschnitt ein streifenförmiger Fortsatz des Prothesenmaterials des Stentgraft-Elements ist.

Wie ebenfalls weiter oben diskutiert, ist gemäß einer weiteren, noch weiter ausgebildeten Ausführungsform des oben erläuterten erfindungsgemäßen Gefäßprothesen-Systems bevorzugt, wenn das Stentgraft-Element und das Stent-Element getrennt voneinander freisetzbar sind, derart, dass das Stent-Element nach dem Stentgraft-Element und zumindest teilweise in diesem freisetzbar ist.

Dabei bedeutet "teilweise in diesem freisetzbar", dass das Stent-Element im expandierten Zustand teilweise, bzw. zu einem größeren Teil in dem Stentgraft-Element platziert ist, wobei ein Teil des Stent-Elements außerhalb des Stentgraft-Elements expandiert ist, und damit der Abschnitt des Gefäßprothesen-Systems darstellt, über welchen Blut durch die ungecoverten Öffnungen des Stent-Elements, bspw. in abzweigende Gefäße, austreten kann.

Allgemein ist bei dem erfindungsgemäßen Gefäßprothesen-System bevorzugt, wenn das Prothesenmaterial ein Material aufweist, das ausgewählt ist aus einem Textil oder einem Polymer.

Insbesondere ist bevorzugt, wenn das Prothesenmaterial ein Material aufweist oder aus diesem gebildet ist, das ausgewählt ist aus Polyester, Polyurethan, Polystyrol, Polytetrafluorethylen ultrahochmolekulargewichtiges Polyethylen (UHMPE), oder Mischungen davon.

Dabei können sowohl das Prothesenmaterial des Stentgraft-Elements, der streifenförmige Prothesenmaterial-Abschnitt und der stentfreie Prothesen-Abschnitt aus einem solchen Material gebildet sein, oder ein solches aufweisen.

Gemäß einer besonders bevorzugten Ausführungsform weist das Stentgraft-Element eine Länge von zwischen ca. 20 mm und ca.100 mm auf, sowie das nicht-gecoverte Stent-Element eine Länge von zwischen ca. 30 mm und ca. 100 mm.

Die Erfindung betrifft ferner die Verwendung eines erfindungsgemäßen Gefäßprothesen-Systems zur Behandlung von Dissektionserkrankungen oder aneurysmatischen Erkrankungen der thorakalen Aorta.

Ferner betrifft die vorliegende Erfindung auch ein Verfahren zur Freisetzung des erfindungsgemäßen Gefäßprothesen-Systems, wobei das Verfahren die folgenden Schritte aufweist:
- Einbringen des Gefäßprothesen-Systems in eine Aorta eines Patienten im komprimierten Zustand, derart, dass das Stentgraft-Element vollständig distal der Arteria subclavia positioniert wird;
- Überführen des Stentgraft-Elements in den expandierten Zustand durch Rückziehen einer das Stentgraft-Element komprimierende ersten Hülle;
- Überführen des Stent-Elements in den expandierten Zustand, durch Rückziehen einer das Stent-Element komprimierenden zweiten Hülle;
- derart, dass das von Prothesenmaterial freie Stent-Element im Bereich der Abgänge des Truncus brachiocephalus, der Arteria carotis communis, und der Arteria subclavia sinistra im Aortenbogen freigesetzt wird, und
- wobei der Prothesen-Abschnitt proximal des Abgangs des Truncus brachiocephalus positioniert wird.

Mit diesem Verfahren wird sicher gestellt, dass das erfindungsgemäße Gefäßprothesen-System derart positioniert wird, dass das nicht-gecoverte Stent-Element den Blutfluss in die abgehenden Gefäße Truncus brachiocephalus, Arteria carotis communis, und Arteria subclavia sinistra ermöglicht.

Hierbei wird darauf geachtet, dass das gecoverte Stentgraft-Element knapp distal des Arteria subclavia Abgangs endet. Das nicht gecoverte Stent-Element wird nun im Aortenbogen freigesetzt, wobei die Drahtmaschen bzw. die Öffnungen des Lasergeschnittenen Stent-Elements so weiträumig sind, dass keine Gefährdung im Sinn einer Verlegung der Abgänge der Kopf-Hals Gefäße (Truncuns brachiocephalicus, linke Aorta carotis communis, und linke Arteria subclavia besteht. Im Anschluss daran wird kragenförmige Prothesen-Abschnitt mit dem proximalen Aortenbogen vernäht werden.

Das erfindungsgemäße Gefäßprothesen-System und das Verfahren zur Einbringung bzw. Freisetzung bieten daher den Vorteil, dass ein im Bereich der Aorta ascendens vorliegender Einriss oder ein Aneurysma durch Resektion und konventionellen prothetischen Einsatz wie bisher beseitigt werden kann, und simultan eventuell verbleibende Einrisse der Intima in der proximalen Aorta descendens oder im Aortenbogen sicher stabilisieren werden können, und zwar ohne Rupturgefahr im Langzeitverlauf. Damit kann insbesondere im Falle einer Dissektionserkrankung oder komplexen aneurysmatischen Erkrankung der thorakalen Aorta der operative und zeitliche Aufwand gegenüber bisher im Stand der Technik angewandten Operationen und Systemen bei einem vergleichbaren Ergebnis deutlich verringert, und damit das Risiko des Eingriffs signifikant gesenkt werden.

Weitere Vorteile ergeben sich aus den Figuren und der nachstehenden Beschreibung bevorzugter Ausführungsbeispiele.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung einer Ausführungsform einer erfindungsgemäßen Gefäßprothese, im nicht-eingeführten; expandierten Zustand;
- Fig. 2: eine schematische Darstellung der in Fig. 1 gezeigten Ausführungsform in einer anderen Perspektive, ebenfalls im nicht-eingeführten, aber expandierten Zustand;
- Fig. 3: eine schematische Darstellung der in Fig. 1 und Fig. 2 gezeigten Ausführungsform, wobei das Stent-Element teilweise durch eine Stent-Element-Hülle komprimiert ist,
- Fig. 4: eine schematische Darstellung der in den Fig. 1 bis 3 gezeigten Ausführungsform, wobei hier das komprimierte Stent-Element zur Ladung auf einen Katheter teilweise innerhalb des Stentgraft-Elements positioniert ist, wobei das Ende des Stent-Elements, das den stentfreien Prothesen-Abschnitt trägt, bereits teilweise freigesetzt ist,
- Fig.5: eine schematische Darstellung des vollständig freigesetzten und expandierten Gefäßprothesen-Systems in ungebogener Form des Stent-Elements,
- Fig. 6: eine schematische Darstellung des vollständig freigesetzten und expandierten Gefäßprothesen-Systems mit einem gebogenem Stent-Element-Abschnitt, und zwar außerhalb eines Gefäßes, und
- Fig. 7: eine schematische Darstellung des vollständig in der Aorta freigesetzten und expandierten Gefäßprothesen-Systems.

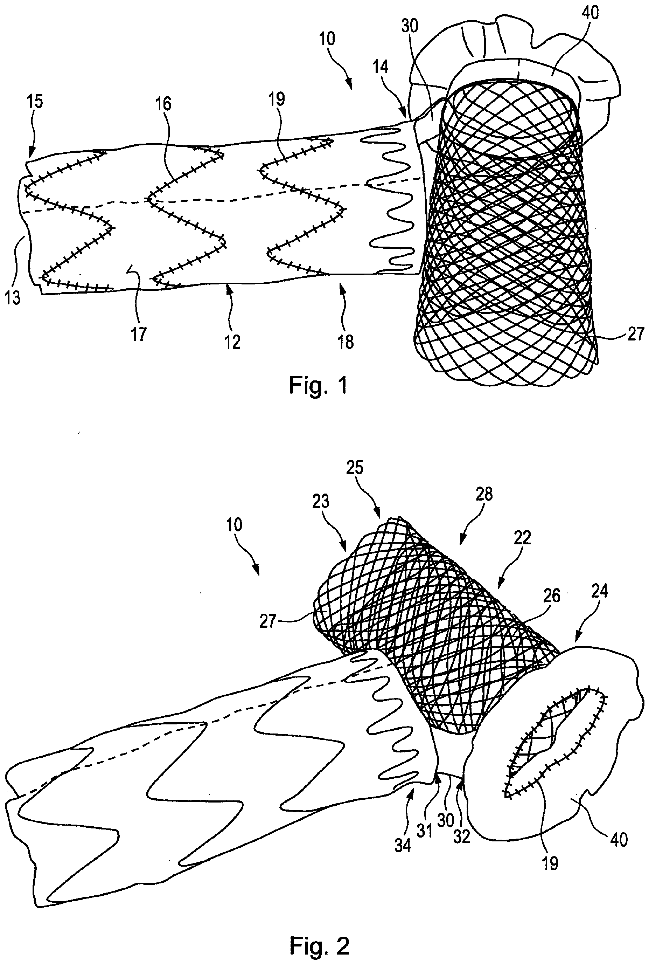

In den Figuren werden gleiche Merkmale mit gleichen Bezugszeichen versehen, wobei aus Gründen der Übersichtlichkeit nicht in allen Figuren immer sämtliche Bezugszeichen angegeben sind. In Fig. 1 und 2 ist mit 10 insgesamt ein Gefäßprothesen-System bezeichnet, mit einem Stentgraft-Element 12 mit einem hohlzylindrischen Körper 13, einem ersten, proximalen Stentgraft-Element-Ende 14 und einem zweiten, distalen Stentgraft-Element-Ende 15. Das Stentgraft-Element 12 weist ferner mäanderförmig umlaufende Stützen 16 und ein an den Stützen 16 befestigtes und diese verbindendes Prothesenmaterial 17 auf, wodurch ein umfänglich gecovertes Stenftgraft-Element 18 gebildet wird. Das Prothesenmaterial 17 ist vorzugsweise an die Stützen 16 angenäht, was durch Nähte 19 angedeutet ist. Die Nähte 19 sind dabei nur in Fig. 1 angedeutet.

Das Gefäßprothesen-System 10 weist ferner ein Stent-Element 22 auf, mit einem hohlzylindrischen Körper 23 und einem ersten proximalen Stent-Element-Ende 24, einem zweiten distalen Stent-Element-Ende 25. Das Stent-Element 22 weist ein Stent-Stützgerüst 26 auf, das frei von Prothesenmaterial ist, wodurch ein ungecovertes Stent-Element 28 gebildet wird, mit Öffnungen bzw. Maschen oder Löchern 27.

Fig. 1 und 2 ist ferner zu entnehmen, dass das Stentgraft-Element 12 und Stent-Element 22 zwei getrennte Baueinheiten sind. Lediglich ein streifenförmiger Prothesenmaterial-Abschnitt 30 verbindet die beiden Elemente 12 und 22, wobei der Prothesenmaterial-Abschnitt 30 zwei Enden, ein erstes Ende 31 und ein zweites Ende 32 aufweist, und wobei der Abschnitt 30 mit seinem ersten Ende 31 lediglich am ersten, proximalen Stentgraft-Element-Ende 14 fixiert ist, und mit seinem zweiten Ende 32 am ersten proximalen Stent-Element-Ende 24. Dadurch wird eine Prothesenmaterial-Brücke 34 ausgebildet wird, die sich vom ersten, proximalen Stentgraft-Element-Ende 14 bis zum ersten, proximalen Stent-Element-Ende 24 erstreckt.

Fig. 1 und 2 ist ferner zu entnehmen, dass das Stent-Element 22 an seinem ersten proximalen Ende 24 umlaufend an diesem einen stentfreien Prothesen-Abschnitt 40 aufweist, der mit dem proximalen Stent-Element-Ende 24 verbunden ist, vorzugsweise angenäht. Dieser stentfreie Prothesen-Abschnitt 40 bildet eine Art Kragen, welcher im eingesetzten Zustand an die Gefäßwand angenäht werden kann.

Auch der streifenförmige Prothesenmaterial-Abschnitt 30 ist über entsprechende Nähte 19 an dem proximalen Stentgraft-Element-Ende 14 einerseits und an dem proximalen Stent-Element-Ende 24 andererseits angenäht.

In dem in Fig. 1 und 2 gezeigten Zustand sind die beiden Elemente 12 und 22 im Verhältnis zueinander praktisch frei beweglich, und nur über die Brücke 34 flexibel, d.h. beweglich, miteinander verbunden.

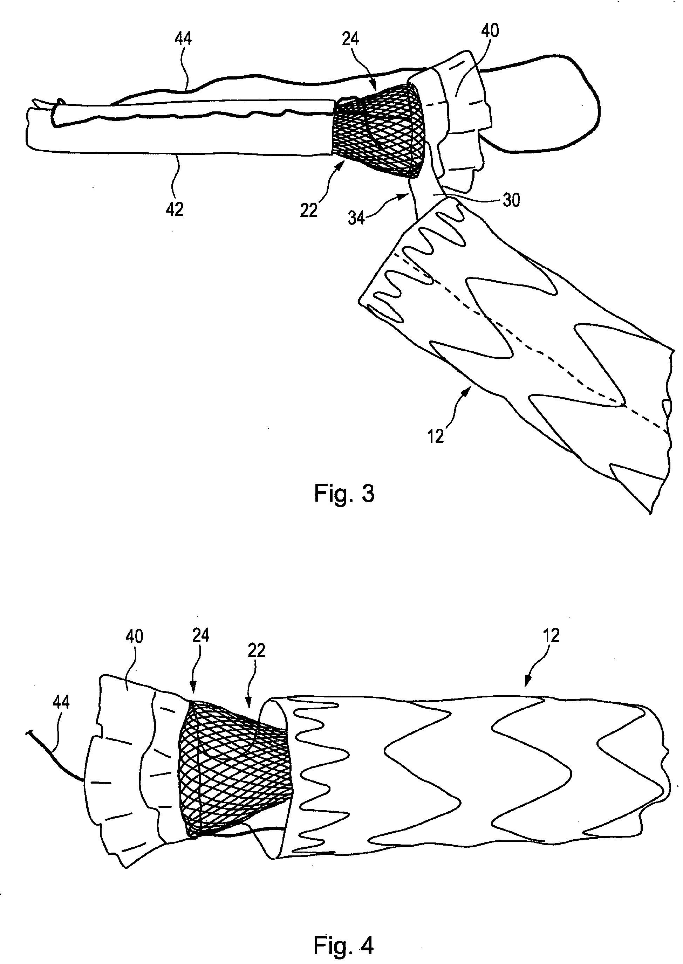

In Fig. 3 ist schließlich gezeigt, dass das Stent-Element 22 durch Überziehen einer Stent-Element-Hülle 42 fast über seine ganze Länge komprimiert ist, lediglich das proximale Stent-Element-Ende 24, an dem sich der stentfreie Prothesen-Abschnitt 40 und ein Ende des streifenförmigen Prothesenmaterial-Abschnitts 30 befindet, ist noch nicht komprimiert. Ebenfalls gezeigt ist ein Faden 44, über welchen die Hülle 42 durch Zugkraft wieder vom Stent-Element 22 abgezogen werden kann, wodurch dieses freigesetzt wird. Die Stent-Element-Hülle 42 kann dabei vorzugsweise aus einem Textilmaterial sein.

Zum Laden auf einen Einführkatheter (nicht gezeigt), werden die beiden Elemente 12 und 22 konzentrisch auf diesen geladen. Dabei liegt das Stent-Element 22 komprimiert innerhalb des Stentgraft-Elements 12 vor, welches Stentgraft-element 12 wiederum von einer Stentgraft-Element-Hülle (nicht gezeigt) komprimiert gehalten wird.

Zum Freisetzen des Gefäßprothesen-Systems wird zunächst die Stentgraft-Element-Hülle zurückgezogen, wodurch diese im Gefäß expandieren, und sich an die Gefäßwände anlegen, bzw. gegen diese mittels Radialkraft drücken kann. In einem nächsten Schritt wird dann die Stent-Element-Hülle 42 durch Zug am Faden 44 entfernt, wodurch das Stent-Element 22 freigesetzt, uns bspw. im Aortenbogen in eine Biegung gezwungen wird. Über den kragenartigen stentfreien Prothesen-Abschnitt 40 wird die Gefäßprothese 10 dann mit einer Naht an der Gefäßwand fixiert.

In Fig. 4 ist die teilweise Freisetzung des Stent-Elements 22 gezeigt: Das proximale Ende 24 des Stent-Elements 22 ist vollständig freigesetzt, wohingegen sich ein größerer Abschnitt noch im komprimierten Zustand innerhalb des Stentgraft-Elements 12 befindet.

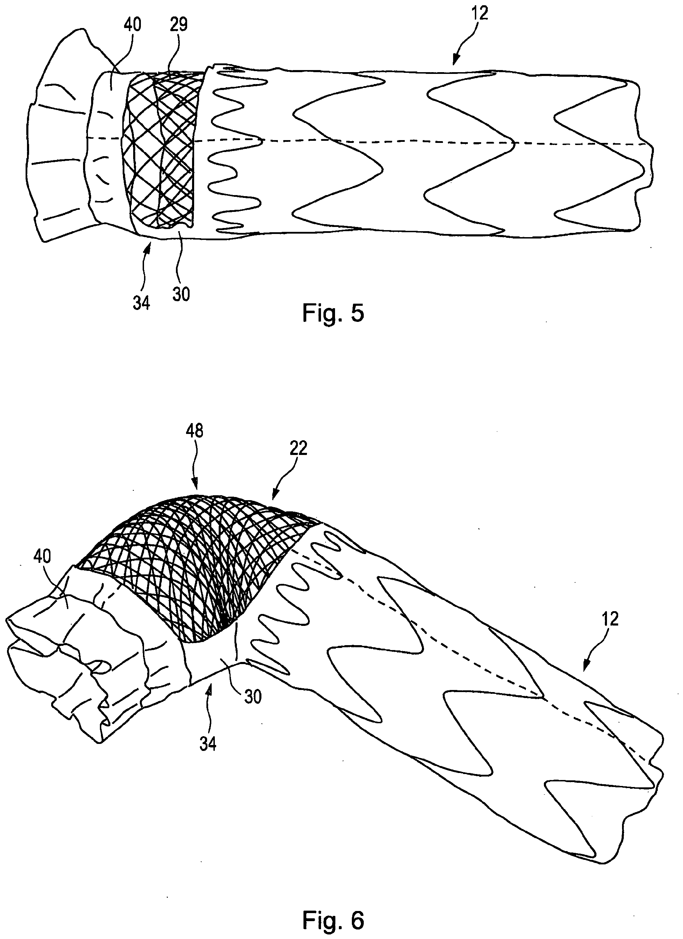

In Fig. 5 ist das Stent-Element 22 schließlich vollständig freigesetzt, und das Stent-Element 22 drückt über seine Radialkraft nach außen, und damit an den Stellen, an denen es sich innerhalb des Stentgraft-Elements 12 befindet, von innen gegen das Stentgraft-Element 12. Gleichzeitig ist ein Teil des Stent-Elements 22 nicht innerhalb des Stentgraft-Elements 12, was durch das Bezugszeichen 29 angedeutet ist.

Wird nun das Stent-Element 22 teilweise aus dem Stentgraft-Element 12 herausgezogen, wird es durch die Prothesenmaterial-Brücke 34 in eine Biegung 48 gezwungen. Dieser Zustand ist in Fig. 6 gezeigt.

Fig. 6 ist auch zu entnehmen, dass - bezogen auf die Krümmungs-/Biegungsachse bzw. Krümmungs-/Biegungsmittelpunkt, die Brücke 34 radial innen zu liegen kommt und die offenen, unbedeckten Löcher 27 zumindest im größeren, radial außen gelegenen Biegungsbereich.

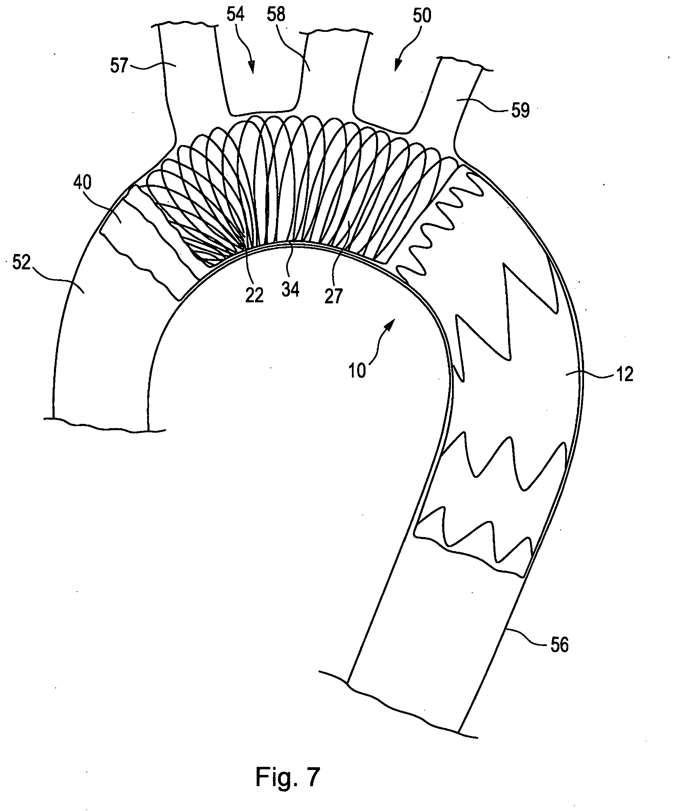

Fig. 7 zeigt schließlich die in den Fig. 1 bis 5 gezeigte Ausführungsform des erfindungsgemäßen Gefäßprothesen-Systems in einem in eine Aorta 50 eingebrachten Zustand: In Fig. 7 ist mit 52 ein Teil der aufsteigenden - ascendierenden - Aorta bezeichnet, mit 54 der Aortenbogen und mit 56 die absteigende - descendierende - Aorta. Wie aus Fig. 7 ersichtlich, gehen im Bereich des Aortenbogens 54 drei Gefäße 57, 58, und 59 ab, nämlich der Truncus brachiocephalus 57, die Arteria carotis communis 58, und die Arteria subclavia sinistra 59.

Fig. 7 ist ferner die Lage bzw. Positionierung der in den Fig. 1 bis 5 dargestellten Ausführungsform des erfindungsgemäßen Gefäßprothesen-Systems 10 zu entnehmen: Zu erkennen ist, dass das Stentgraft-Element 12, bzw. dessen distales Ende 15, proximal des Truncus brachiocephalus beginnt, dass ferner das von Prothesenmaterial freie Stent-Element 22 im Aortenbogen 54 freigesetzt ist und über seine Zellen bzw. Öffnungen oder Maschen 27 die abgehenden Gefäße 57, 58 und 59 mit Blut versorgen kann, und dass schließlich der stentfreie Prothesen-Abschnitt 40 distal der Arteria subclavia sinistra 59 beginnt.

Zur Einführung der erfindungsgemäßen Gefäßprothese 10 wird diese, wie weiter oben erwähnt, auf ein Einführsystem geladen (nicht gezeigt) und zwar derart, dass die beiden Elemente 12 und 22 konzentrisch auf diesem und mit jeweils separaten und separaten zu bedienenden Rückzugshüllen in einem komprimierten Zustand gehalten. Verfahren und Vorrichtungen zum Einführen von Gefäßprothesen sind dem Fachmann aus dem Stand der Technik geläufig.

Die komprimiert gehaltene Gefäßprothese 10 wird in die descendierende Aorta vorgeschoben, und die korrekte Platzierung kann bspw. über entsprechende, an der Gefäßprothese 10 vorgesehene - bspw. röntgendichte - Marker kontrolliert werden. Nach korrekter Platzierung wird dann zunächst das Stentgraft-Element 12 durch Rückziehen der Stentgraft-Element-Hülle freigesetzt, und danach das Stent-Element 22 durch Rückziehen der Stent-Element-Hülle 42, welches Stent-Element 22 als nicht-gecoverter Gefäßprothesenabschnitt im Aortenbogen 54 freigesetzt wird, wobei die Öffnungen bzw. Maschen 27 so weiträumig sind, dass keine Gefährdung im Sinn einer Verlegung der Abgänge der Kopf-Hals Gefäße 57, 58 und 59 (Truncuns brachiocephalicus, linke Aorta carotis communis, linke Arteria subclavia) besteht.

Abschließend wird proximal des Abgangs des Truncus brachiocephalicus 57 der stentfreie Prothesen-Abschnitt mit dem proximalen Aortenbogen vernäht.

Dem Fachmann wird klar sein, dass er die genauen Maße und räumlichen Anforderungen der einzelnen Gefäßprothesen-Elemente 12, 22 und 40 sowie die Länge der Prothesenmaterial-Brücke 34 durch vorherige Untersuchung des zu behandelnden Patienten ermitteln und jeweils spezifisch umsetzen kann.

## Patentansprüche

1. Gefäßprothesen-System (10) zur Einführung in und Unterstützung eines Blutgefäß eines Patienten, welches Gefäßprothesen-System (10) von einem komprimierten Zustand in einen expandierten Zustand überführbar ist, und wobei das Gefäßprothesen-System (10) folgendes aufweist:
ein Stentgraft-Element (12) mit einem hohlzylindrischen Körper (13), einem ersten, proximalen Stentgraft-Element-Ende (14) und einem zweiten, distalen Stentgraft-Element-Ende (15), wobei das Stentgraft-Element (12) mäanderförmig umlaufenden Stützen (16) und ein an den Stützen (16) befestigtes und diese verbindendes Prothesenmaterial (17) aufweist zur Ausbildung eines umfänglich gecoverten Stentgraft-Elements (18), und
ein Stent-Element (22) mit einem hohlzylindrischen Körper (23), einem ersten, proximalen Stent-Element-Ende (24) und einem zweiten, distalen Stent-Element-Ende (25), wobei das Steht-Element (22) ein Stent-Stützgerüst (26) aufweist, das frei von Prothesenmaterial ist, zur Ausbildung eines ungecoverten Stent-Elements (28),
**dadurch gekennzeichnet, dass**
das Stentgraft-Element (12) und das Stent-Element (22) zwei baulich voneinander getrennte Elemente des Gefäßprothesen-Systems (10) sind, und wobei das Stent-Element (22) derart dimensioniert und ausgebildet ist, dass es über sein zweiten, distalen Stent-Element-Ende (25) zumindest teilweise über das erste, proximale Stentgraft-Element-Ende (14) in dieses einführbar und in diesem expandierbar ist, wobei ferner das Stent-Element (22) an seinem ersten, proximalen Ende (24) umlaufend mit einem stentfreien Prothesen-Abschnitt (40) fest verbunden ist, welcher Prothesen-Abschnitt (40) lediglich aus Prothesenmaterial (18) besteht,
und wobei ferner das Stentgraft-Element (12) und das Stent-Element (22) nur über einen streifenförmigen Prothesenmaterial-Abschnitt (30) miteinander verbunden sind, derart, dass der streifenförmige Prothesenmaterial-Abschnitt (30) mit einem ersten Ende (31) am ersten, proximalen Stentgraft-Element-Ende (14) und mit seinem zweiten Ende (32) am ersten, proximalen Stent-Element-Ende (24) zur Ausbildung einer Prothesenmaterial-Brücke (34) fixiert ist.

2. Gefäßprothesen-System (10) nach Anspruch 1, **dadurch gekennzeichnet, dass** der stentfreie Prothesen-Abschnitt (40) am ersten proximalen Stent-Element-Ende (24) kragenförmig ausgebildet ist.

3. Gefäßprothesen-System (10) nach Anspruch 1 oder 2, **dadurch gekennzeichnet, dass** es zur Implantation in eine Aorta (50), insbesondere im Bereich der aufsteigenden Hauptschlagader (52), des Aortenbogens (54) und der absteigenden Hauptschlagader (56) ausgebildet ist.

4. Gefäßprothesen-System (10) nach einem der Ansprüche 1 bis 3, **dadurch gekennzeichnet, dass** zumindest die Stützten (16) des Stentgrafts-Elements (12) und/oder das Stent-Stützgerüst (26) des Stent-Elements (22) aus einem selbst-expandierenden Material sind oder ein solches aufweisen.

5. Gefäßprothesen-System (10) nach einem der Ansprüche 1 bis 4, **dadurch gekennzeichnet, dass** das von Prothesenmaterial freie Stent-Element (22) im expandierten Zustand im Bereich des Aortenbogens (54) freisetzbar ist.

6. Gefäßprothesen-System (10) nach einem der Ansprüche 1 bis 5, **dadurch gekennzeichnet, dass** das Stentgraft-Element (12) zwischen zwei und acht hintereinander angeordnete Stützen (16) aufweist.

7. Gefäßprothesen-System (10) nach einem der Ansprüche 1 bis 6, **dadurch gekennzeichnet, dass** das von Prothesenmaterial freie Stent-Element (22) ein geflochtenes, gezwirbeltes oder Laser-geschnittenes Stent-Stützgerüst (26) aufweist.

8. Gefäßprothesen-System (10) nach einem der Ansprüche 1 bis 7, **dadurch gekennzeichnet, dass** der streifenförmige Prothesenmaterial-Abschnitt (30) das Stentgraft-Element (12) und das Stent-Element (22) nur auf einem Kreisbogen von kleiner gleich 180° verbindet.

9. Gefäßprothesen-System (10) nach einem der Ansprüche 1 bis 8, **dadurch gekennzeichnet, dass** der streifenförmigen Prothesenmaterial-Abschnitt (30) ein streifenförmiger Fortsatz des Prothesenmaterials (17) des Stentgraft-Elements (12) ist.

10. Gefäßprothesen-System (10) nach einem der Ansprüche 1 bis 9, **dadurch gekennzeichnet, dass** das Stentgraft-Element (12) und das Stent-Element (22) getrennt voneinander freisetzbar sind, derart, dass das Stent-Element (22) nach dem Stentgraft-Element (12) und zumindest teilweise in diesem freisetzbar ist.

## Claims

1. A vascular prosthesis system (10) for inserting into and supporting a blood vessel of a patient, which vascular prosthesis system (10) is convertible from a compressed state to an expanded state, said vascular prosthesis system (10) having the following:
a stent graft element (12) with a hollow cylindrical body (13), a first, proximal stent graft element end (14) and a second, distal stent graft element end (15), the stent graft element (12) having supports (16) extending circumferentially in a meandering formation and having a prosthesis material (17) attached to the supports (16) thereby connecting them, for forming a circumferentially covered stent graft element (18), and
a stent element (22) with a hollow cylindrical body (23), a first, proximal stent element end (24) and a second, distal stent element end (25), the stent element (22) having a stent support framework (26), which is free of prosthesis material, for forming an uncovered stent element (28),
**characterized in that**
the stent graft element (12) and the stent element (22) are two structurally separate elements of the vascular prosthesis system (10), and that the stent element (22) is dimensioned and designed, such, that it is insertable with its second, distal stent element end (25) at least partially into the first, proximal stent graft element end (14) and is expansible therein,
wherein furthermore the stent element (22), at its first, proximal end (24), is firmly connected circumferentially to a stent-free prosthesis portion (40), which prosthesis portion (40) is made solely of prosthesis material (18),
and wherein furthermore the stent graft element (12) and the stent element (22) are connected to each other only by a strip-shaped prosthesis material portion (30), such, that the strip-shaped prosthesis material portion (30) is fixed with a first end (31) on the first, proximal stent graft element end (14) and is fixed with its second end (32) on the first, proximal stent element end (24) in order to form a prosthesis material bridge (34).

2. The vascular prosthesis system (10) as claimed in claim 1, **characterized in that** the stent-free prosthesis portion (40) is formed in the shape of a collar on the first, proximal stent element end (24).

3. The vascular prosthesis system (10) as claimed in claim 1 or 2, **characterized in that** it is designed for implantation in an aorta (50), particularly in the area of the ascending aorta (52), the aortic arch (54) and the descending aorta (56).

4. The vascular prosthesis system (10) as claimed in one of claims 1 through 3, **characterized in that** at least the supports (16) of the stent graft element (12) and/or the stent support framework (26) of the stent element (22) are made from a self-expanding material or have such a material.

5. The vascular prosthesis system (10) as claimed in one of claims 1 through 4, **characterized in that** the stent element (22) free of prosthesis material is releasable in the expanded state in the area of the aortic arch (54).

6. The vascular prosthesis system (10) as claimed in one of claims 1 through 5, **characterized in that** the stent graft element (12) has between two and eight successive supports (16).

7. The vascular prosthesis system (10) as claimed in one of claims 1 through 6, **characterized in that** the stent element (22) free of prosthesis material has a braided, twisted or laser-cut stent support framework (26).

8. The vascular prosthesis system (10) as claimed in one of claims 1 through 7, **characterized in that** the strip-shaped prosthesis material portion (30) connects the stent graft element (12) and the stent element (22) only over an arc of a circle of less than or equal to 180°.

9. The vascular prosthesis system (10) as claimed in one of claims 1 through 8, **characterized in that** the strip-shaped prosthesis material portion (30) is a strip-shaped continuation of the prosthesis material (17) of the stent graft element (12).

10. The vascular prosthesis system (10) as claimed in one of claims 1 through 9, **characterized in that** the stent graft element (12) and the stent element (22) are releasable separately from each other, in such a way that the stent element (22) is releasable after the stent graft element (12) and at least partially within the latter.

## Revendications

1. Système de prothèse vasculaire (10) pour introduire dans et soutenir un vaisseau sanguin d'un patient, système de prothèse vasculaire (10) qui peut être amené d'un état comprimé à un état dilaté, et dans lequel le système de prothèse vasculaire (10) comprend:
un élément d'endoprothèse couverte (12) avec un corps cylindrique creux (13), une première extrémité proximale d'élément d'endoprothèse couverte (14) et une seconde extrémité distale d'élément d'endoprothèse couverte (15), dans lequel l'élément d'endoprothèse couverte (12) présente des segments périphériques sinueux (16) et un matériau de prothèse (17) fixé aux segments (16) et reliant ceux-ci pour la formation d'un élément d'endoprothèse couvert en périphérie (18), et
un élément d'endoprothèse (22) avec un corps cylindrique creux (23), une première extrémité proximale d'élément d'endoprothèse (24) et une seconde extrémité distale d'élément d'endoprothèse (25), dans lequel l'élément d'endoprothèse (22) présente une structure de soutien d'endoprothèse (26), qui est libre de matériau de prothèse, pour la formation d'un élément d'endoprothèse non couvert (28),
**caractérisé en ce que**
l'élément d'endoprothèse couverte (12) et l'élément d'endoprothèse (22) sont des éléments structurellement séparés l'un de l'autre du système d'endoprothèse vasculaire (10), et dans lequel l'élément d'endoprothèse (22) est dimensionné et configuré de telle manière qu'il puisse être introduit par sa seconde extrémité distale d'élément d'endoprothèse (25) au moins partiellement par la première extrémité proximale de l'élément d'endoprothèse couverte (14) dans celui-ci et puisse être dilatée dans celui-ci,
dans lequel l'élément d'endoprothèse (22) est en outre solidement relié par sa première extrémité proximale (24) en périphérie à une partie de prothèse sans endoprothèse (40), partie de prothèse (40) qui se compose uniquement de matériau de prothèse (18),
et dans lequel l'élément d'endoprothèse couverte (12) et l'élément d'endoprothèse (22) ne sont en outre reliés l'un à l'autre que par une partie de matériau de prothèse en forme de bande (30), de telle manière que la partie de matériau de prothèse en forme de bande (30) soit fixée par une première extrémité (31) à la première extrémité proximale d'élément d'endoprothèse ouverte (14) et par sa seconde extrémité (32) à la première extrémité proximale d'élément d'endoprothèse (24) pour la formation d'un pont de matériau de prothèse (34).

2. Système de prothèse vasculaire (10) selon la revendication 1, **caractérisé en ce que** la partie de prothèse sans endoprothèse (40) est réalisée en forme de collet à la première extrémité distale (24) de l'élément d'endoprothèse.

3. Système de prothèse vasculaire (10) selon la revendication 1 ou 2, **caractérisé en ce qu'**il est configuré en vue d'une implantation dans une aorte (50), en particulier dans la région de l'aorte ascendante (52), de l'arc aortique (54) et de l'aorte descendante (56).

4. Système de prothèse vasculaire (10) selon l'une quelconque des revendications 1 à 3, **caractérisé en ce qu'**au moins les segments (16) de l'élément d'endoprothèse couverte (12) et/ou la structure de soutien d'endoprothèse (26) de l'élément d'endoprothèse (22) sont réalisés en matériau auto-expansible ou qui en présentent.

5. Système de prothèse vasculaire (10) selon l'une quelconque des revendications 1 à 4, **caractérisé en ce que** l'élément d'endoprothèse sans matériau de prothèse (22) peut être libéré dans l'état dilaté dans la région de l'arc aortique (54).

6. Système de prothèse vasculaire (10) selon l'une quelconque des revendications 1 à 5, **caractérisé en ce que** l'élément d'endoprothèse couverte (12) présente entre deux et huit segments (16) disposés l'un derrière l'autre.

7. Système de prothèse vasculaire (10) selon l'une quelconque des revendications 1 à 6, **caractérisé en ce que** l'élément d'endoprothèse sans matériau de prothèse (22) présente une structure de soutien d'endoprothèse (26) tressée, torsadée ou coupée au laser.

8. Système de prothèse vasculaire (10) selon l'une quelconque des revendications 1 à 7, **caractérisé en ce que** la partie de matériau de prothèse en forme de bande (30) relie l'élément d'endoprothèse couverte (12) et l'élément d'endoprothèse (22) uniquement sur un arc de cercle de moins de 180°.

9. Système de prothèse vasculaire (10) selon l'une quelconque des revendications 1 à 8, **caractérisé en ce que** la partie de matériau de prothèse en forme de bande (30) est un prolongement en forme de bande du matériau de prothèse (17) de l'élément d'endoprothèse couverte (12) .

10. Système de prothèse vasculaire (10) selon l'une quelconque des revendications 1 à 9, **caractérisé en ce que** l'élément d'endoprothèse couverte (12) et l'élément d'endoprothèse (22) peuvent être libérés séparément l'un de l'autre, de telle manière que l'élément d'endoprothèse (22) puisse être libéré après l'élément d'endoprothèse couverte (12) et au moins partiellement dans celui-ci.
